# FASCICULE DE BREVET EUROPEEN

(11) **EP 1 204 714 B1**
(45) Date de publication et mention de la délivrance du brevet: **28.01.2004**
(21) Numéro de dépôt: 00949662.1
(22) Date de dépôt: 06.07.2000
(51) Int. Cl.: C09K 9/02, G02B 5/23, C07D 311/92

(54) **COMPOSES PHOTOCHROMIQUES NAPHTO[2,1-B]PYRANES A SUBSTITUTIONS BI OU TERTHIENYLE**
BI- ODER TERTHIENYL-SUBSTITUIERTE PHOTOCHROMISCHE NAPHTHO[2,1-B]PYRANE-VERBINDUNGEN
BI-OR TERTHIENYL SUBSTITUTED PHOTOCHROMIC NAPHTHO 2,1-B]PYRAN COMPOUNDS

(30) Priorité: 08.07.1999 FR 9908875
(43) Date de publication de la demande: 15.05.2002
(73) Titulaire: ESSILOR INTERNATIONAL COMPAGNIE GENERALE D'OPTIQUE, 94227 Charenton cédex (FR)
(72) Inventeur: FRIGOLI, Michel, F-13009 Marseille (FR); MOUSTROU, Corinne, F-13008 Marseille (FR); SAMAT, André, F-13013 Marseille (FR); GUCLIELMETTI, Robert, F-13009 Marseille (FR)
(74) Mandataire: Catherine, Alain
(86) Numéro de dépôt international: PCT/FR2000/001954
(87) Numéro de publication internationale: WO 2001/004233

(56) Documents cités:
- EP-A- 0 835 870
- WO-A-98/45281
- US-A- 5 651 923
- US-A- 5 808 100
- MOUSTROU, CORINNE ET AL: "Synthesis of thiophene-substituted 3H-naphtho[2,1-b]pyrans, precursors of photomodulated materials" HELV. CHIM. ACTA (1998), 81(7), 1293-1302, XP002132847

## Description

L'invention a pour objet des composés photochromiques, plus particulièrement des composés hétérocycliques de la famille des naphto[2,1-b]pyranes substitués par des groupements bi ou terthiényle, ainsi que leur application dans le domaine des matériaux et articles à transmission optique variable.

Quand des composés photochromiques sont soumis à une irradiation contenant des rayonnements ultraviolets (soleil, lampes xénon ou mercure), ils subissent un changement de couleur réversible. Dès que l'excitation cesse, ils reviennent à leur couleur originelle.

Ces dernières années, les matériaux organiques destinés à des applications optiques ont fait l'objet de nombreuses recherches. Les verres ophtalmiques, les verres de bâtiment, les pare-brise de voitures ou d'avions, les visières de casques, dont la transparence dans le visible peut être modulée par l'utilisation de molécules photochromiques, ont plus particulièrement retenu l'attention. Pour ce type d'application utilisant la lumière solaire (héliochromisme), le composé photochromique actif doit répondre à un certain nombre de critères, parmi lesquels :
- une forte colorabilité dans le visible après excitation par la lumière (la colorabilité est la mesure de la capacité pour un composé photochromique de présenter une couleur intense);
- une absence de coloration (ou une faible coloration) à l'état initial;
- une cinétique rapide de décoloration thermique à température ambiante;
- une faible décoloration par la lumière visible; et
- une vitesse de coloration élevée.

Une des difficultés majeures rencontrées avec les composés photochromiques est l'obtention d'un compromis entre une forte colorabilité et une cinétique rapide de décoloration. En effet, sous irradiation solaire continue, un équilibre photostationnaire s'établit entre les molécules qui se colorent sous l'action de la lumière ultraviolette et celles qui se décolorent sous l'action conjuguée de la température et de la lumière visible. Ainsi, fréquemment, une augmentation de la vitesse de décoloration entraîne une diminution de la colorabilité.

La société demanderesse a découvert une nouvelle famille de naphto[2,1-b]pyranes substitués par des groupements bithiényle ou terthiényle présentant des propriétés photochromiques particulièrement intéressantes.

Les composés conformes à l'invention présentent, par rapport aux naphto[2,1-b]pyranes de l'art antérieur une colorabilité au moins équivalente pour des vitesses de coloration et des vitesses de décoloration plus élevées. On a également constaté pour ces composés :
- une absence de coloration ou une très faible coloration à l'état original ;
- une durée de vie particulièrement longue;
- une sensibilité aux rayonnements ultraviolets de faible énergie (longueurs d'ondes supérieures à 380 nm).
- une absorption des formes colorées à grande longueur d'onde (539 nm et plus).

L'ensemble de ces caractéristiques rend ces nouveaux composés particulièrement intéressants pour la fabrication de matériaux photochromiques, en particulier comprenant un substrat en matériau polymère transparent tel que des verres organiques à transmission optique variable (verres de lunettes solaires, vitrage de bâtiment, pare-brise de voitures ou d'avions, visières de casques de pilote).

Les composés photochromiques peuvent être directement incorporés au substrat en verre organique ou dissous dans un film polymérique acollé au substrat en verre organique.

Les composés photochromiques selon l'invention possèdent une structure [3H]naphto[2,1-b]pyrane et se caractérisent par le fait qu'ils comportent au moins un groupement bithiényle, substitué ou non, dans l'une des positions 5 à 10 du cycle naphtalénique de la structure [3H]naphto[2,1-b]pyrane et au moins un groupement bithiényle ou terthiényle, substitué ou non, en position 3 de ladite structure et à l'exclusion des composés de structure [3H]naphto[2,1-b]pyrane comportant un seul groupement bithiényle en position 3 de ladite structure et un groupement bithiényle en position 7 du cycle naphtalénique.

De préférence, les composés photochromiques selon l'invention comprennent un groupement bithiényle en position 5, 6, 8-10 du cycle naphtalénique.

Plus particulièrement, les composés de l'invention répondent à la formule générale suivante : 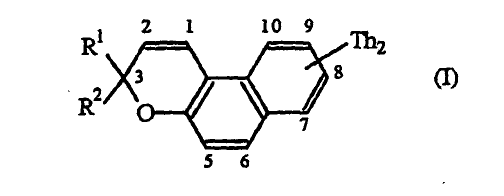 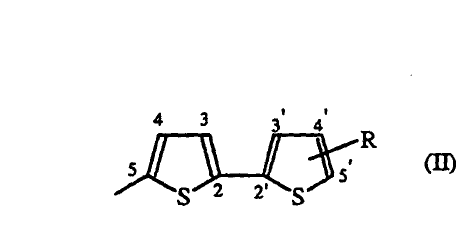 où Th₂ est représenté par la formule (II) :
dans laquelle :
R désigne un substituant pouvant occuper l'une des positions 3, 3', 4, 4' ou 5', préférentiellement 5', et choisi parmi un atome d'hydrogène, un groupement alkyle, un groupement cycloalkyle, un groupement aryle, un groupement OR³, SR³, COR³ ou COOR³ où R³ désigne un atome d'hydrogène, un groupement alkyle ou cycloalkyle, un groupement aryle, un groupement amino, un groupement NO₂, CN ou SCN, un atome d'halogène, un groupement mono ou polyhaloalkyle, 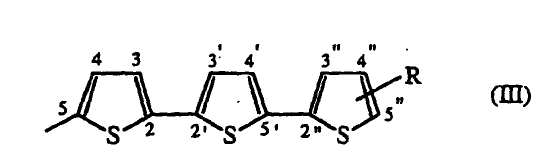 R¹ désigne un groupement
   Th₂ tel que représenté par la formule (II) ou un groupement terthiényle (Th₃) tel que représenté par la formule (III) :
   où R est un substituant tel que désigné ci-dessus pour décrire la formule (II) et pouvant occuper l'une des positions 3, 3', 3", 4, 4', 4", 5", de préférence 5", et
R² peut être un groupement Th₂ tel que défini dans la formule (II), un groupement Th₃ tel que défini dans la formule (III), un groupement aryle, un groupement naphtyle, un groupement thiényle, un groupement furanyle, un groupement pyrrolyle ou N-alkylpyrrolyle.

Dans cette définition de R², un groupement aryle désigne un phényle, un phényle mono ou polysubstitué par un (ou des) substituant(s) alkyle, cycloalkyle, phényle, amino, un halogène, un groupement OR³, SR³, COR³ ou COOR³ (où R³ a la même signification que précédemment); les groupements R² du type naphtyle peuvent être substitués avec les mêmes substituants.

Les groupements hétérocycliques (thiophène, furane, pyrrole) peuvent être benzocondensés et le groupe benzo peut porter des substituants alkyle, cycloalkyle, aryle, OR³, SR³, COR³ ou COOR³ (où R³ a la même signification que précédemment), amino, NO₂, CN ou SCN, mono (ou poly)haloalkyle.

De préférence, R¹ représente un groupe Th₃.

Dans les substituants bithiényles et terthiényles, de préférence, les groupements alkyle sont des groupes alkyle en C₁-C₆, par exemple méthyle, éthyle, propyle, butyle, pentyle et hexyle; les groupements cycloalkyle sont des groupes cycloalkyle en C₅-C₇, par exemple cyclopentyle, cyclohexyle et cycloheptyle; les groupements aryle désignent un phényle, un phényle mono ou polysubstitué par un (ou des) substituant(s) alkyle en C₁-C₆, cycloalkyles en C₅-C₇, phényle et amino; les halogènes représentent de préférence Br, Cl et F; et les groupements mono (ou poly-)haloalkyle sont de préférence des groupes alkyle en C₁-C₆ mono (ou poly) chlorés ou fluorés, par exemple CF₃.

Les composés représentés par la formule (I), peuvent être préparés par la réaction de couplage d'un alcool propargylique (A) convenablement substitué avec un 2-naphtol porteur du groupement bithiényle (B) (schéma 1).

Cette réaction est effectuée dans le dichlorométhane en présence d'un catalyseur acide (PPTS : paratoluène sulfonate de pyridinium) :

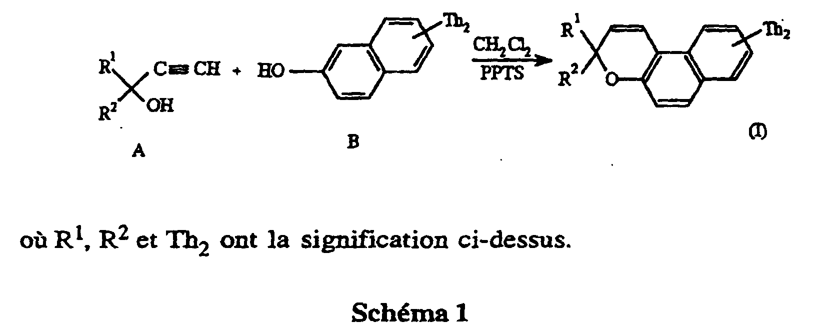

Les alcools propargyliques peuvent être préparés comme décrit dans le schéma 2, par l'action de l'acétylure de lithium sur une cétone convenablement substituée.

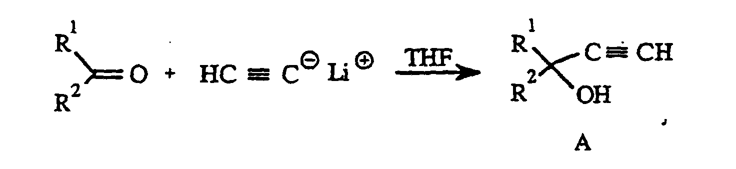

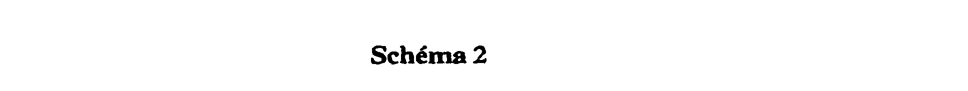

La préparation des naphtols (B) peut être réalisée de deux façons différentes suivant que le groupement bithiényle doit occuper la position 7 ou les autres positions (5, 6, 8-10) sur la molécule cible (I).

La préparation du 5-(2,2'-bithien-5-yl)-2-hydroxynaphtalène 6 (précurseur des naphtopyranes (I) substitués en position 7 par le groupement Th₂) est réalisée en quatre étapes comme indiqué sur le schéma 3. Le bromure de bithiényl magnésium 2 (obtenu par une réaction de transmétallation à partir du 2,2'-bithiophène 1) réagit avec la 6-méthoxy-1-tétralone (3) pour conduire au dérivé 4. Après une réaction d'oxydation en présence de chloranil (2, 3, 5, 6-tétrachloro-1,4-benzoquinone), le composé résultant 5 est déméthylé par le tribromure de bore dans le dichlorométhane pour conduire au naphtol attendu 6.

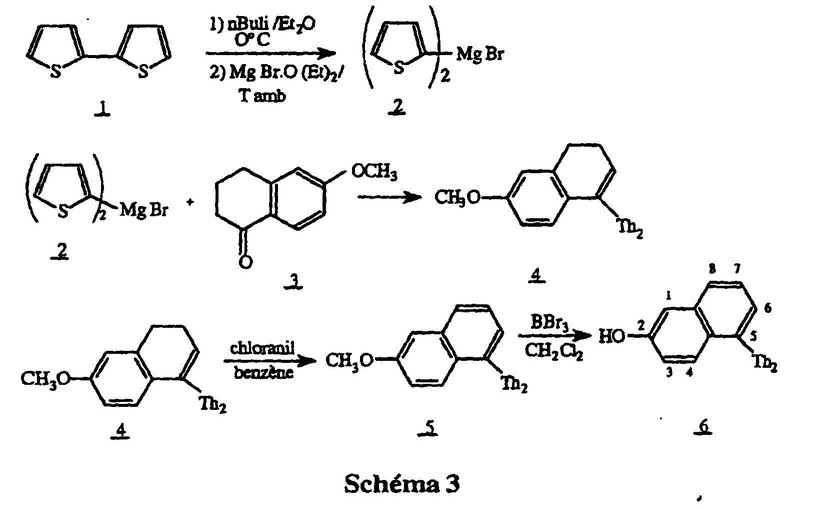

Les naphtols du type 9 (permettant d'accéder aux naphtopyranes de formule (I) substitués en positions 5, 6, 8, 9 ou 10 par un groupement Th₂) sont préparés à partir d'une réaction de couplage catalysée par un catalyseur au palladium tel que Pd (PPh₃)₄ entre le dérivé boronique 7 du bithiophène et un bromo-2-naphtol (schéma 4) ou un 2-naphtol substitué par un groupement triflate, le substituant brome ou groupement triflate occupant l'une des positions 3, 4, 6-8 du cycle naphtalénique.

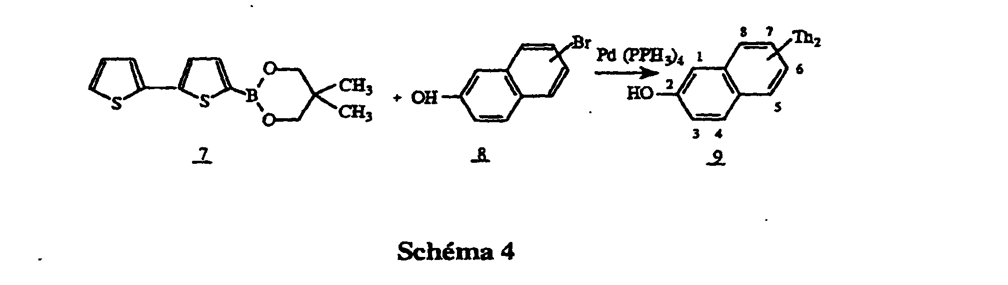

Les naphtopyranes de la présente invention montrent des propriétés photochromiques particulièrement intéressantes comparées à celles de molécules de la même famille de l'art antérieur et en particulier aux [3H]naphto[2,1-b]pyranes ayant un substituant bithiényle en position 7 du cycle naphtalénique. Une solution toluénique des composés de formule (I) se colore et se décolore très rapidement à température ambiante. L'intérêt majeur de ces nouveaux composés est que les réponses rapides à la coloration comme à la décoloration n'entraînent pas de diminution significative de la colorabilité comme on aurait pu s'y attendre et peuvent même quelquefois être accompagnées d'une augmentation ce celle-ci.

Un autre intérêt des composés conformes à l'invention est qu'ils se colorent sous l'action de rayonnements ultraviolets d'une longueur d'onde supérieure à 380 nm alors que la plupart des composés similaires de l'art antérieur se colorent peu ou pas dans les mêmes conditions. Cette propriété rend possible l'utilisation de ce type de composés photochromiques pour la réalisation d'articles transparents en verre organique absorbant les rayonnements ultraviolets de longueurs d'onde plus courtes (vitrage de bâtiment, pare-brise d'avion ou d'automobile), en particulier pour la fabrication de verres de lunettes ou de visières de casques de pilotes.

Les composés conformes à l'invention peuvent être introduits directement dans une matrice polymère transparente ou être incorporés dans une composition destinée à être appliquée sur un matériau polymère organique transparent. Dans ce cas, les composés objet de l'invention, sont dissous dans un solvant approprié (par exemple chloroforme, acétate d'éthyle, acétone, acétonitrile, dichlorométhane, benzène) et incorporés à une solution de polymère (par exemple polyuréthane, polyacrylate, polyméthacrylate) dans le même solvant.

Les compositions sont ensuite appliquées sous forme de film de quelques micromètres d'épaisseur sur un support transparent polymérique (tel que polycarbonate, acétate de cellulose, polyalkylacrylate), pour obtenir un matériau photochromique qui peut se colorer en présence d'un rayonnement ultraviolet et revenir rapidement à l'état non coloré et transparent en l'absence de source de lumière.

Les composés conformes à l'invention présentent l'intérêt de permettre ce changement de coloration un grand nombre de fois à des températures voisines de l'ambiante.

Plus précisément, les composés conformes à l'invention peuvent être introduits dans une composition destinée à être appliquée sur ou être introduite dans un matériau polymère organique transparent pour obtenir un article transparent photochromique. Ils peuvent également être introduits dans des compositions solides telles que films plastiques, plaques et lentilles pour réaliser des matériaux utilisables notamment comme lentilles ophtalmiques, lunettes de soleil, viseurs, optique de caméra et filtres, vitrages de bâtiment, pare-brise d'avion ou d'automobile et visières de casques.

Les compositions liquides qui constituent un objet de l'invention sont essentiellement caractérisées par le fait qu'elles contiennent sous forme dissoute ou dispersée les composés conformes à l'invention dans un milieu à base de solvants appropriés pour être appliqués ou introduits dans un matériau polymère transparent.

Des solvants plus particulièrement utilisables sont des solvants organiques choisis parmi le benzène, le toluène, le chloroforme, le dichlorométhane, l'acétate d'éthyle, la méthyléthylcétone, l'acétone, l'alcool éthylique, l'alcool méthylique, l'acétonitrile, le tétrahydrofurane, le dioxane, l'éther méthylique d'éthylèneglycol, le diméthylformamide, le diméthylsulfoxyde, le méthylcellosolve, la morpholine et l'éthylène glycol.

Lorsque les composés conformes à l'invention sont dispersés, le milieu peut également contenir de l'eau.

Selon une autre forme de réalisation, les composés conformes à l'invention peuvent être introduits et de préférence dissous dans des solutions incolores ou transparentes préparées à partir de polymères, de copolymères ou de mélanges de polymères transparents dans un solvant organique approprié.

Parmi les polymères et copolymères, on peut citer les polyuréthanes, les poly(méth)acrylates, les poly allyl(méth)acrylates, les dérivés de cellulose, tels que la nitrocellulose, l'acétate de cellulose et l'éthylcellulose, le polychlorure de vinyle, le polystyrène, le poly(alkyl) styrène, et la polyvinylpyrrolidone.

Des exemples de telles solutions sont entre autres des solutions de nitrocellulose dans l'acétonitrile, de polyvinylacétate dans l'acétone, de chlorure de polyvinyle dans la méthyléthylcétone, de polyméthylméthacrylate dans l'acétone, d'acétate de cellulose dans le diméthylformamide, de polyvinylpyrrolidone dans de l'acétonitrile, de polystyrène dans le benzène, d'éthylcellulose dans du chlorure de méthylène.

Ces compositions peuvent être appliquées sur des supports transparents tels qu'en térèphtalate de polyéthylèneglycol, de papier borylé, de triacétate de cellulose et séchées pour obtenir un matériau photochromique qui peut se colorer en présence d'une radiation ultraviolette, et qui retourne à l'état non coloré et transparent en l'absence de la source de radiation.

Les composés photochromiques de la présente invention ou les compositions les contenant définies ci-dessus peuvent être appliqués ou incorporés dans un matériau organique polymérisé transparent solide approprié pour des articles transparents tels que des lentilles ophtalmiques ou des matériaux utiles pour être utilisés dans des lunettes de soleil, des viseurs, des optiques de caméras et des filtres, des vitrages de bâtiment, pare-brise d'avion ou d'automobile et visières de casques.

A titre de matériaux solides transparents qui peuvent être utilisés pour réaliser des lentilles ophtalmiques conformes à l'invention, on peut citer les polymères de polyol(allylcarbonate), des polyacrylates, des poly(alkylacrylate) tels que des polyméthylméthacrylates, l'acétate de cellulose, le triacétate de cellulose, le propionate acétate de cellulose, le butyrate acétate de cellulose, le poly(vinylacétate), le poly(vinyl alcool), les polyuréthanes, les polycarbonates, les polyéthylène térèphtalates, les polystyrènes, les (polystyrène-méthyl-méthacrylate), les copolymères de styrène et d'acrylonitrile, les polyvinylbutyrates.

Les copolymères transparents ou des mélanges de polymères transparents sont également appropriés pour réaliser de tels matériaux.

On peut citer à ce sujet les matériaux préparés à partir de polycarbonates tels que le poly(4,4'-dioxydiphénol-2,2 propane), le polyméthylméthacrylate, les polyol(allylcarbonate) tels qu'en particulier le diéthylèneglycol bis(allylcarbonate) et ses copolymères tels que par exemple avec l'acétate de vinyle. On peut citer en particulier les copolymères de diéthylèneglycol bis(allylcarbonate) et d'acétate de vinyle (80-90/10-20) et encore le copolymère de diéthylèneglycol bis(allylcarbonate) avec l'acétate de vinyle, l'acétate de cellulose et le propionate de cellulose, le butyrate de cellulose (80-85/15-20).

Les polyols(allylcarbonate) sont préparés en utilisant des allyl carbonates de polyols liquides aliphatiques ou aromatiques, linéaires ou ramifiés, tels que les glycols aliphatiques de bis-allylcarbonate ou les alkylène bis(allylcarbonate). Parmi les polyols (allylcarbonate) qui peuvent être utilisés pour préparer les matériaux transparents solides utilisables conformément à l'invention, on peut citer l'éthylène glycol bis(allylcarbonate), le diéthylèneglycol bis(2-méthallylcarbonate), le diéthylèneglycol bis(allylcarbonate), l'éthylèneglycol bis(2-chloro allylcarbonate), le triéthylèneglycol bis(allylcarbonate), le 1,3-propane diol bis(allylcarbonate), le propylèneglycol bis(2-éthylallylcarbonate), le 1,3-butanediol bis(allylcarbonate), le 1,4-butanediol bis(2-bromo-allylcarbonate), le dipropylèneglycol bis(allylcarbonate), le triméthylèneglycol bis(2-éthylallylcarbonate), le pentaméthylèneglycol bis(allylcarbonate), l'isopropylène bisphénol bis (allylcarbonate). Le produit le plus important est constitué par le diéthylèneglycol bis(allyl carbonate) encore connu sous la dénomination CR39.

La quantité de composés photochromiques à utiliser conformément à l'invention, soit dans la composition, soit au moment de son introduction dans le support solide, n'est pas critique et dépend généralement de l'intensité de la couleur que la composition peut conférer au matériau après exposition aux radiations. D'une manière générale, plus on ajoute de composés photochromiques, plus la coloration sous irradiation sera importante.

Conformément à l'invention, on utilise une quantité suffisante pour conférer au matériau traité la propriété de changer de couleur au moment de l'exposition à la radiation. Cette quantité de composés photochromiques est généralement comprise entre 0,01 et 20% en poids et de préférence entre 0,05 et 10% en poids par rapport au poids total du matériau optique ou de la composition.

Les composés photochromiques conformes à l'invention peuvent également être introduits dans un support temporaire (tel qu'un vernis formant un revêtement sur un substrat) de transfert et être transférés ensuite thermiquement dans le substrat comme décrit en particulier dans le brevet US-4 286 957 ou US-4 880 667.

Ces composés peuvent être utilisés avec d'autres composés photochromiques, tels que des composés photochromiques donnant lieu à des colorations différentes telles que bleu, verte, connus dans l'état de la technique. C'est ainsi qu'on peut utiliser des spiro(indoline-oxazines) bien connus dans l'état de la technique.

Les exemples suivants sont destinés à illustrer l'invention sans pour autant présenter un caractère limitatif.

Dans les exemples, sauf indication contraire, tous les pourcentages et parties sont exprimés en poids.

### Exemple comparatif 1

### Synthèse du 3,7-di(2,2'-bithien-5-yl)-3-(thien-2-yl)-[3H]-naphto[2,1-b] pyrane.

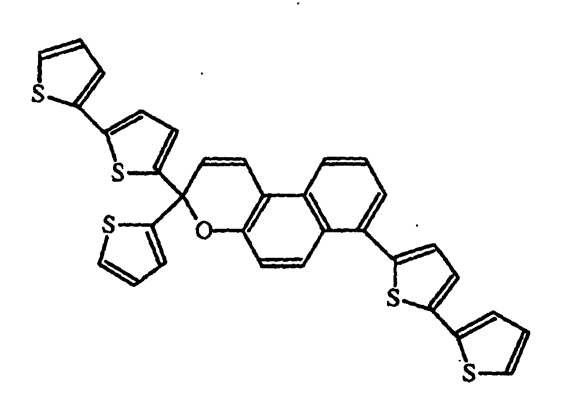

### Etape 1

Dans un ballon bicol de 250 ml muni d'un système d'agitation surmonté d'un réfrigérant, on introduit 0,072 mole de 2-bromothiophène et 2 g de [1,3-bis(diphényl phosphino)-propane] dichloronickel (II) dissous préalablement dans 100 ml d'éther éthylique anhydre. Le milieu réactionnel est maintenu sous argon. Du bromure de 2-thiényl magnésium fraîchement préparé (0,072 mole) est additionné à 0°C, l'addition dure environ 40 minutes. Le mélange réactionnel est agité pendant 3 heures. On refroidit au bain de glace et une solution aqueuse d'acide sulfurique à 10% est additionnée lentement jusqu'à l'obtention de deux phases limpides. Après décantation, la phase aqueuse est extraite avec de l'éther éthylique (3 x 100 ml). Les phases organiques regroupées sont séchées sur sulfate de magnésium.

Après évaporation du solvant sous pression réduite, le produit brut est purifié par chromatographie sur colonne de silice, éluê avec du pentane pur.

Le 2,2'-bithiophène est obtenu avec un rendement de 95%.
Formule brute : C₈H₆S₂
Masse moléculaire : 166,220
Point de fusion : 32°C.

### Etape 2

Sous argon, un mélange constitué de 6 mmoles de chlorure de 4-méthoxybenzoyle, 6 mmoles de 2,2'-bithiophène préparé selon l'étape 1 et 15 ml de benzène anhydre, est refroidi à l'aide d'un bain d'eau glacée. 6 mmoles de SnCl₄ sont additionnées lentement. Le mélange est agité pendant 30 minutes. La formation d'un précipité est observée, 30 ml de benzène, puis 15 ml d'une solution aqueuse d'acide chlorhydrique à 10% sont ajoutés. Dès que la dissolution du précipité est complète, la phase aqueuse est extraite au benzène. La phase organique résultante est lavée à l'eau jusqu'à l'obtention d'un pH neutre, séchée sur sulfate de magnésium puis concentrée sous pression réduite. La (2,2'-bithien-5-yl) (thien-2-yl) cétone résultante est lavée au pentane puis filtrée.
Formul brute : C₁₃H₈S₃
Masse molaire : 276,404 g
Rendement : 83%
Point de fusion : 98°C.

### Etape 3

Sous argon, un mélange constitué d'acétylure de lithium (15 mmoles) et 200 ml de THF anhydre, est refroidi à 0°C à l'aide d'un bain eau-glace. 1,5 mmoles de la cétone préparée dans l'étape 2 sont alors ajoutées en une seule fois. Après être revenu à température ambiante, le mélange réactionnel est agité pendant 3 heures. La réaction est suivie par chromatographie sur couche mince de silice. La solution est alors hydrolysée avec une solution aqueuse saturée en chlorure d'ammonium. Le mélange eau-THF est extrait à l'éther éthylique.

Les phases organiques résultantes sont réunies, séchées sur sulfate de magnésium, puis concentrées sous pression réduite. Le produit brut est chromatographié sur gel de silice avec des mélanges pentane/dichlorométhane de polarité croissante (50 : 50 à 0 : 100). Le 1-(2,2'-bithien 5-yl)-1-(thien-2-yl) prop-2-yn-1-ol est isolé sous forme d'huile.
Rendement : 81 %
Formule brute : C₁₅H₁₀OS₃.
Masse molaire : 302,441.

### Etape 4

Dans un ballon tricol de 250 ml muni d'un système d'agitation surmonté d'un réfrigérant, on introduit 0,018 moles de bithiophène préparé dans l'étape 3 dissous dans 90 ml d'éther éthylique anhydre. Le mélange réactionnel est refroidi à 0°C, puis 0,021 moles de n-butyl lithium 1,6 M dans l'hexane sont ajoutées lentement.

La réaction procède sous argon. Au bout d'une heure, on ajoute 0,018 moles de MgBr.O(Et)₂ sur le dérivé lithié.

Le mélange réactionnel est laissé deux heures à température ambiante puis 18 mmoles de 6-méthoxy-1-tétralone préalablement dissous dans 15 ml d'éther anhydre sont additionnées. On porte au reflux de l'éther éthylique pendant 5 heures. On laisse revenir le mélange réactionnel à température ambiante, puis on refroidit (bain de glace). Une solution aqueuse d'acide sulfurique à 10% est additionnée lentement jusqu'à l'obtention de deux phases limpides. Après décantation, la phase aqueuse est extraite à l'éther éthylique (3 x 100 ml).

Après évaporation du solvant sous pression réduite, le produit brut est purifié par chromatographie sur colonne de silice et élué avec des mélanges constitués de pentane et d'éther éthylique de polarité croissante (100 : 0 à 80 : 20). Le 1-(2,2'-bithien 5-yl)-3,4-dihydro-6-méthoxy-naphtalène est isolé sous forme d'huile.
Rendement : 60%
Formule brute : C₁₉H₁₆OS₂
Masse molaire : 324,467 g.

### Etape 5

Dans un bicol de 250 ml muni d'un système d'agitation surmonté d'un réfrigérant, on introduit 27 mmoles de chloranil et 50 ml de benzène anhydre. La réaction procède sous argon. A l'aide d'une ampoule à brome, on additionne goutte à goutte 9,2 mmoles du cycloalcène obtenu lors de l'étape 4, dissous préalablement dans 20 ml de benzène, le mélange réactionnel est alors porté au reflux du solvant pendant 5 heures. La solution refroidie est filtrée sous vide sur silice et on procède rapidement à une élution à l'éther éthylique. Après évaporation du solvant sous pression réduite, le produit brut est purifié par chromatographie su colonne de silice et élué avec des mélanges constitués de pentane et d'éther éthylique de polarité croissante (100 : 0 à 80 : 20).

Le composé huileux résiduel est le 1-(2,2'-bithien 5-yl)-6-méthoxynaphtalène.
Rendement : 93%
Formule brute : C₁₉H₁₄OS₂
Masse molaire : 422,451 g.

### Etape 6

Dans un tricol de 50 ml muni d'un système d'agitation surmonté d'un réfrigérant, on introduit 8,6 mmoles du composé obtenu lors de l'étape 5 et 15 ml de CH₂Cl₂ préalablement distillé. Le mélange réactionnel est refroidi à 0°C, puis 11,2 ml (11 mmoles) d'une solution 1M de BBr₃ dans CH₂Cl₂ sont additionnés. L'addition dure environ 20 minutes. La réaction procède sous argon. On chauffe ensuite au reflux du CH₂Cl₂ pendant 15 heures. On laisse revenir le mélange réactionnel à température ambiante, puis on neutralise par une solution saturée de Na₂CO₃ jusqu'à l'obtention d'un pH égal à 7. Le mélange obtenu est filtré sur célite. Après décantation, la phase aqueuse est extraite avec CH₂Cl₂ (3 x 10 ml). Les phases organiques sont regroupées, lavées à l'eau, puis séchées sur sulfate de magnésium. Après évaporation du solvant sous pression réduite, le produit brut est purifié par chromatographie sur colonne de silice et élué avec des mélanges constitués de pentane et d'éther éthylique de polarité croissante (100 : 0 à 50 : 50). Le 1-(2,2'-bithien 5-yl) 6-hydroxynaphtalène est isolé avec un rendement de 24%.
Formule brute : C₁₈H₁₂OS₂
Masse molaire : 308,424 g
Point de fusion : 133°C.

### Etape 7

Un mélange constitué de 1 mmole d'alcool propargylique préparé lors de l'étape 3, 1 mmole du naphtol préparé lors de l'étape 6 et 20 ml de CH₂Cl₂ anhydre est agité sous argon.

Une quantité catalytique de paratoluène sulfonate de pyridinium est additionnée à température ambiante. Le mélange réactionnel est laissé sous agitation pendant 6 à 8 heures. La réaction est suivie par chromatographie sur couche mince analytique. Le mélange est versé sur une solution glacée de soude à 5%. La phase aqueuse est extraite trois fois au dichlorométhane. Les phases organiques réunies sont séchées sur sulfate de magnésium puis concentrées sous pression réduite. Le résidu est purifié pa chromatographie sur colonne de silice avec des mélanges pentane/dichlorométhane de polarité croissante (100: 0 à 50: 50). Le 3,7-di(2,2'-bithien-5-yl)-3-(thien-2-yl)[3H]-naphto[2,1-b]pyrane est isolé avec un rendement de 30%.
Formule brute : C₃₃H₂₀OS₅
Point de fusion : 98°C.

### Exemple 1

### Synthèse du 3,8-di(2,2'-bithien-5-yl)-3-(4-méthoxyphényl)-[3H]-naphto [2,1-b]pyrane

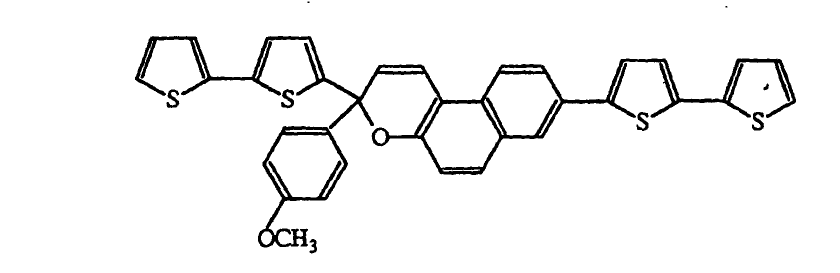

### Etape 1

La (2,2'-bithien-5-yl)(4-méthoxyphényl)cétone est préparée suivant le même mode opératoire que celui décrit dans l'étape 2 de l'exemple comparatif 1. Les précurseurs sont ici le chlorure de l'acide 4-méthoxybenzoïque et le 2,2'-bithiophène.
Rendement : 85%
Formule brute : C₁₆H₁₂O₂S₂
Masse molaire : 300,402 g
Point de fusion : 119-120°C.

### Etape 2

Le 1-(2,2'-bithien-5-yl)-1-(4-méthoxyphényl)prop-2-yn-1-ol est préparé suivant le même mode opératoire que celui décrit dans l'étape 3 de l'exemple comparatif 1. Le précurseur est ici la cétone décrite ci-dessus (étape 1, exemple 1).
Rendement : 87%
Formule brute : C₁₈H₁₄O₂S₂
Masse molaire : 326,441 g
Produit huileux.

### Etape 3

Dans un ballon tricol, sous argon, on introduit 6 mmoles de 2,2'-bithiophène dilué dans 30 ml de THF anhydre. Le mélange est refroidi à - 40°C. 2,65 ml (1,05 mmoles) d'une solution 2,5M de nBuli dans l'hexane sont alors ajoutés lentement. Le mélange résultant est agité pendant 1 heure à -40°C, puis refroidi à -90°C. 4 ml (18 mmoles) de B(OBu)₃ sont alors additionnés le plus rapidement possible. Le mélange est laissé revenir à température ambiante puis 30 mmoles de 2,2-diméthyl propane-1,3-diol sont alors ajoutées. Le mélange résultant est agité pendant 10 minutes puis hydrolysé avec 30 ml d'eau. Le mélange eau-THF est extrait au benzène (3 x 30 ml). La phase organique résultante est lavée plusieurs fois avec une solution aqueuse saturée en NaCl, séchée sur sulfate de magnésium puis concentrée sous pression réduite. Le brut chromatographié sur colonne de silice, élué avec des mélanges pentane/dichlorométhane de polarité croissante (100 : 0 à 70 : 30), conduit au 5,5-diméthyl-2-(2,2'-bithien-2-yl)[1,3,2]dioxoborinane.
Rendement : 46%
Formule brute : C₁₃H₂₅O₂S₂B
Masse molaire : 278,20 g

### Etape 4

Un mélange de 3,59 mmoles de l'ester boronique préparé lors de l'étape 3 (exemple 1), 3 mmoles de 6-bromo-2-hydroxynaphtalène, 4,5 mmoles de K₃PO₄, une quantité catalytique de Pd (PPh₃)₄ et 9 ml de DMF est porté à 100°C pendant 24 heures. On laisse le mélange réactionnel revenir à température ambiante, puis on dilue avec 20 ml de THF. La solution est filtrée sur célite. Le THF est évaporé sous pression réduite. Par addition d'eau, les produits organiques précipitent. On filtre et le solide résultant est recristallisé dans un mélange heptane/THF.

Le 2-(2,2'-bithien-5-yl)-6-hydroxynaphtalène est obtenu avec un rendement de 78%.
Formule brute : C₁₈H₁₂OS₂
Masse molaire : 308,329 g
Point de fusion : 231°C.

### Etape 5

Le 3,8-di(2,2-bithien-5-yl)-3-(4-méthoxyphényl)-[3H]-naphto [2,1-b]pyrane est obtenu selon le mode opératoire décrit dans l'étape 7 de l'exemple comparatif 1. Les précurseurs sont ici l'alcool propargylique et le naphtol décrits respectivement dans les étapes 2 et 4 de l'exemple 1. Le produit final est recristallisé dans le toluène.
Rendement : 72%
Formule brute : C₃₆H₂₄O₂S₄
Masse molaire : 616,658 g
Point de fusion : 210°C.

### Exemple 2

### Synthèse du 3,8-di(2,2'-bithien-5-yl)-3-(thien-2-yl)-[3H]-naphto[2,1-b] pyrane

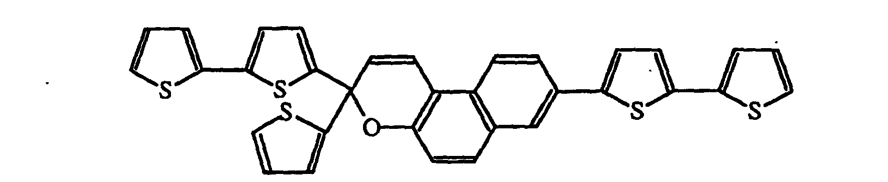

### Etape 1

La (2,2'-bithien-5-yl)-(thien-2-yl)cétone est obtenue à partir du chlorure de l'acide 2-thiophène carboxylique et du 2,2'-bithiophène en suivant le mode opératoire de l'étape 2 de l'exemple comparatif 1. Rendement : 83%
Formule brute: C₁₃H₈OS₄
Masse molaire : 276,404 g
Point de fusion : 98°C.

### Etape 2

Le 1-(2,2'-bithien-5-yl)-1(thien-2-yl)prop-2-yn-1-ol est obtenu à partir de la cétone décrite dans l'étape 1 (exemple 2) suivant le mode opératoire décrit dans l'étape 3 de l'exemple comparatif 1.
Rendement : 81 %
Formule brute : C₃₅H₁₀OS₃
Masse molaire : 302,441 g
Produit huileux.

### Etape 3

Le 3,8-di(2,2'-bithien-5-yl)-3-(thien-2-yl)-[3H]-naphto[2,1-b] pyrane est obtenu suivant le mode opératoire décrit dans l'étape 7 de l'exemple comparatif 1. Les précurseurs sont ici l'alcool propargylique décrit dans l'étape 2 ci-dessus (exemple 2) et le naphtol décrit dans l'étape 4 de l'exemple 1. Le produit final est recristallisé dans le toluène.
Rendement : 67%
Formule brute : C₃₃H₂₀OS₅
Masse molaire : 592,692 g
Point de fusion : 213°C.

### Exemple 3

### Synthèse du 3,8-di(2,2'-bithien-5-yl)3-(4-pentyloxyphényl)-[3H] naphto[2,1-b]pyrane

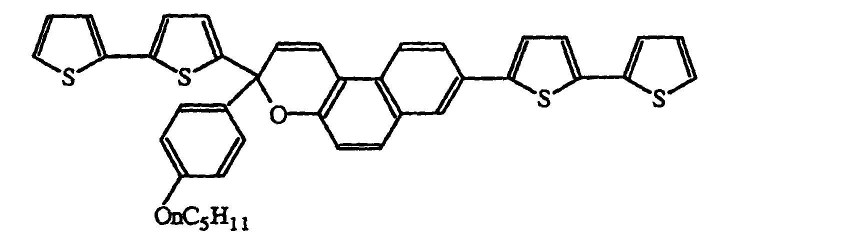

### Etape 1

La (2,2'-bithien-5-yl)(4-pentyloxyphényl)cétone est obtenue à partir du chlorure de l'acide 4-pentyloxybenzoïque et du 2,2'-bithiophène en suivant le mode opératoire de l'étape 2 de l'exemple comparatif 1.
Rendement : 93%
Formule brute : C₂₀H₂₀O₂S₂
Masse molaire : 356,350 g
Point de fusion : 114°C.

### Etape 2

Le 1-(2,2'-bithien-5-yl)-1-(4-pentyloxyphényl)prop-2-yn-1-ol est obtenu à partir de la cétone décrite dans l'étape 1 (exemple 4) suivant le mode opératoire décrit dans l'étape 3 de l'exemple comparatif 1.
Rendement : 81%
Formule brute : C₁₅H₁₀OS₃
Masse molaire : 302,441 g
Produit huileux.

### Etape 3

Le 3,8-di(2,2'-bithien-5-yl)-3-(4-pentyloxyphényl)-[3H]-naphto[2,1-b]pyrane est obtenu suivant le mode opératoire décrit dans l'étape 7 de l'exemple comparatif 1. Les précurseurs sont ici l'alcool propargylique décrit dans l'étape 2 ci-dessus (exemple 3) et le naphtol décrit dans l'étape 4 de l'exemple 1. Le produit final est recristallisé dans l'heptane.
Rendement : 78%
Formule brute : C₄₀H₃₂O₂S₄
Masse molaire : 672,702
Point de fusion : 147°C.

### Exemple 4

### Synthèse de 3,3,8-tri(2,2'-bithien-5-yl)-[3H]-naphto[2,1-b]pyrane

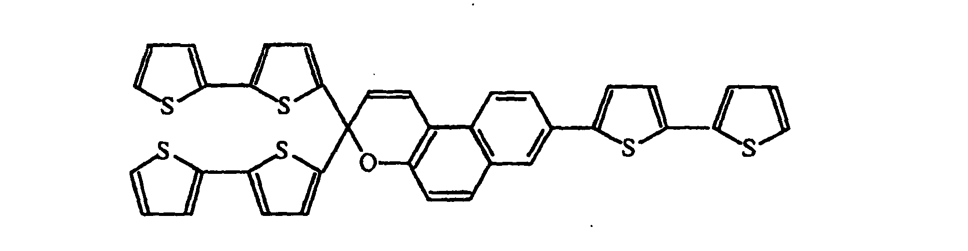

### Etape 1

La di(2,2'-bithien-5-yl)cétone est obtenue à partir du chlorure de l'acide 5-(2'-thiényl)-2-thénoïque et du 2,2'-bithiophène en suivant le mode opératoire de l'étape 2 de l'exemple comparatif 1. La purification est effectuée par chromatographie sur colonne de silice en utilisant des mélanges pentane/CH₂Cl₂ de polarité croissante (0 : 100 à 50 : 50) comme éluant. Une recristallisation est effectuée dans le benzène.
Rendement : 66%
Formule brute : C₁₇H₁₀OS₄
Masse molaire : 358,450 g
Point de fusion : 181°C.

### Etape 2

Le 1-di(2,2'-bithien-5-yl)prop-2-yn-1-ol est obtenu à partir de la cétone décrite dans l'étape 1 (exemple 4) suivant le mode opératoire décrit dans l'étape 3 de l'ex ...Àl'exemple comparatif 1. Il est utilisé sans purification pour l'étape suivante, une chromatographie sur silice ou alumine dégradant ce composé.
Formule brute : C₁₉H₁₂OS₄
Masse molaire : 384,472 g
Produit huileux.

### Etape 3

Le 3,3,8-tri(2,2'-bithien-5-yl)-[3H]-naphto[2,1-b]pyrane est obtenu suivant le mode opératoire décrit dans l'étape 7 de l'exemple comparatif 1. Les précurseurs sont ici l'alcool propargylique décrit dans l'étape 2 ci-dessus (exemple 4) et le naphtol décrit dans l'étape 4 de l'exemple 1. Le produit final est recristallisé dans le toluène.
Rendement : 44% (Etape 2 + Etape 3)
Formule brute : C₃₇H₂₂OS₆
Masse molaire : 674,802 g
Point de fusion : 223°C.

### Exemple 5

### Synthèse du 3,8-di(2,2'-bithien-5-yl)-3-(5-méthoxythien-2-yl)-[3H]-naphto[2,1-b]pyrane

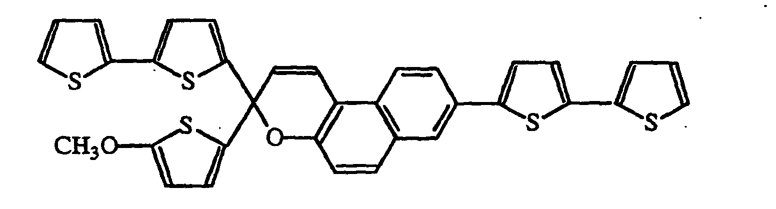

### Etape 1

La (2,2'-bithien-5-yl)(5-méthoxythien-2-yl)cétone est obtenue à partir du chlorure de l'acide 5-méthoxy-thénoïque et du 2,2'-bithiophène en suivant le mode opératoire de l'étape 2 de l'exemple comparatif 1. La purification est effectuée par chromatographie sur une colonnede silice en utilisant des mélanges de pentane/CH₂Cl₂ de polarité croissante comme éluants.
Rendement : 72 %
Formule brute : C₁₄H₁₀O₂S₃
Masse molaire : 306,350 g
Point de fusion : 106°C.

### Etape 2

Le 1-(2,2'-bithien-5-yl)-1-(5-méthoxythien-2-yl)prop-2-yn-1-ol est obtenu à partir de la cétone décrite dans l'étape 1 (exemple 5) suivant le mode opératoire décrit dans l'étape 3 de l'exemple comparatif 1. Il est utilisé sans purification pour l'étape suivante, une chromatographie sur silice dégradant le composé.
Formule brute : C₁₆H₁₂O₂S₃
Masse molaire : 322,374 g
Produit huileux.

### Etape 3

Le 3,8-di(2,2'-bithien-5-yl)-3-(5-méthoxythien-2-yl)-[3H]-naphto[2,1-b]pyrane est obtenu suivant le mode opératoire décrit dans l'étape 7, de l'exemple comparatif 1. Les précurseurs sont ici l'alcool propargylique décrit dans l'étape 2 (exemple 5) et le naphtol décrit dans l'étape 4 de l'exemple 1. Le produit final est recristallisé dans le toluène.
Rendement : 52% (Etape 2 + Etape 3)
Formule brute : C₃₄H₂₂O₂S₅
Masse molaire : 622,702 g
Point de fusion : 204°C.

### Exemple 6

### Synthèse du 8- (2,2'-bithien-5-yl)-3-(4-pentyloxyphényl)-3-(2,2'-5',2"-terthien-5-yl)-[3H]-naphto[2,1-b]pyrane

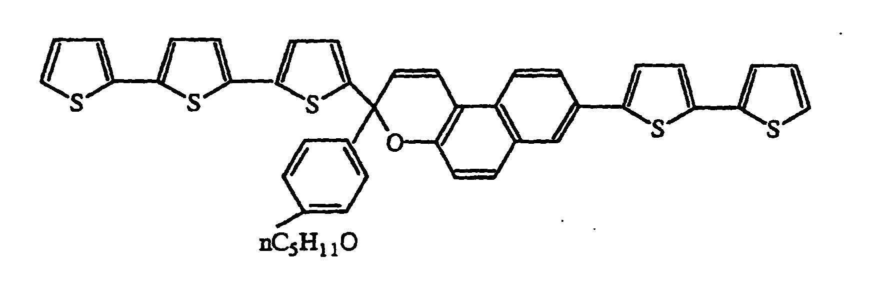

### Etape 1

La (4-pentyloxyphényl)(2,2'-5',2"-terthien-5-yl)cétone est obtenue à partir du chlorure de l'acide 4-pentyloxybenzoïque et du 2,2'-5',2"-terthiophène en suivant le mode opératoire de l'étape 2 de l'exemple comparatif 1. La purification est effectuée par chromatographie sur colonne de silice en utilisant des mélanges pentane/CH₂Cl₂ de polarité croissante comme éluants.
Rendement : 53%
Formule brute : C₂₄H₂₂O₂S₃
Masse molaire : 438,460 g
Point de fusion : 174°C.

### Etape 2

Le 1-(4-pentyloxyphényl)-1-(2,2'-5',2"-terthien-2-yl)prop-2-yn-1-ol est obtenu à partir de la cétone décrite dans l'étape 1 (exemple 6) suivant le mode opératoire décrit dans l'étape 3 de l'exemple comparatif 1.
Rendement : 73%
Formule brute : C₂₇H₂₄O₂S₃
Masse molaire : 478,275 g
Produit huileux.

### Etape 3

Le 8-(2,2'-bithien-5-yl)-3-(4-pentyloxyphényl)-3-(2,2'-5',2"-terthien-5-yl)[3H]naphto[2,1-b]pyrane est obtenu suivant le mode opératoire décrit dans l'étape 7 de l'exemple comparatif 1. Les précurseurs sont ici l'alcool propargylique décrit dans l'étape 2 ci-dessus (exemple 6) et le naphtol décrit dans l'étape 4 de l'exemple 1. Le produit final est recristallisé dans le toluène.
Rendement : 63%
Formule brute : C₄₄H₃₅O₂S₅
Masse molaire : 755,812 g
Point de fusion : 158°C.

### Exemple comparatif 2

Le 3,3'-diphényl[3H]naphto[2,1-b]pyrane est préparé de la façon suivante:

10 mmoles de 1,1-diphényl-2-propyn-1-ol et 11 mmoles de 2-naphtol sont dissous à chaud dans le toluène anhydre (20 ml). On ajoute une quantité catalytique d'acide paratoluène sulfonique. On porte au reflux sous argon pendant 2 h 30 minutes. On laisse revenir à température ambiante et on rajoute 20 ml d'une solution aqueuse de NaOH à 5%. On extrait la phase aqueuse en continu au chlorure de méthylène. Les phases organiques sont réunies et séchées sur sulfate de magnésium. Le solvant est évaporé sous pression réduite. Le produit résultant est purifié par lavage à l'hexane.
Rendement : 62%
Formule brute : C₂₄H₁₈O
Masse molaire : 334,42 g
Point de fusion : 158°C.

### Exemple comparatif 3

Le 3,3-di(4-méthoxyphényl)[3H]naphto[2,1-b]pyrane est préparé de la façon suivante:

Le mode opératoire est identique à celui utilisé dans l'exemple comparatif 2, le précurseur dans ce cas étant le 1,1-di(4-méthoxyphényl)-2-propyn-1-ol.

### Exemple 7

### Synthèse du 3,5-di(2,2'-bithien-5-yl-3-(4-pentyloxyphényl)-[3H]-naphto[2,1-b]pyrane

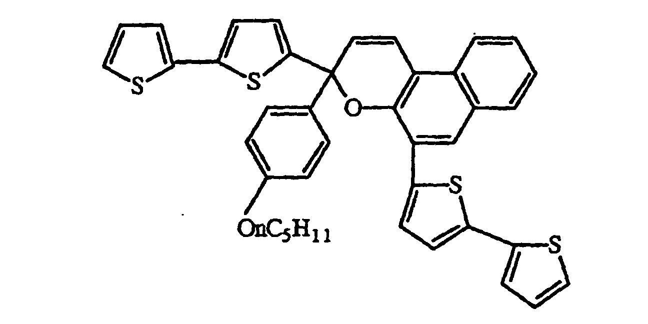

### Etape 1

La (2,2'-bithien-5-yl)-4-pentyloxyphényl cétone a été obtenue suivant le même protocole que celui décrit lors de l'étape 1 de l'exemple 3.

### Etape 2

Le 1-(2,2'-bithien-5-yl)-1-(4-pentyloxyphényl)prop-2-yn-1-ol a été obtenu suivant le même protocole que celui décrit lors de l'étape 2 de l'exemple 3.

### Etape 3

Sous argon, 12 mmoles de dihydroxynaphtalène et 25 ml de pyridine sont laissés sous agitation à température ambiante pendant 20 minutes. Le mélange est refroidi à 0°C et 2,1 ml (12 mmoles) de l'anhydride trifluorométhane sulfonique sont introduits lentement.

Le mélange est laissé revenir à température ambiante et agité toute la nuit. La solution est ensuite versée sur de l'eau glacée (25 ml), puis extraite à l'éther éthylique (3 x 30 ml). La phase organique résultante est lavée à l'eau (2 x 20 ml), puis avec une solution aqueuse saturée en sel (20 ml). La phase organique est séchée sur sulfate de magnésium. Après évaporation sous pression réduite, on isole une huile de couleur brune. Le brut est chromatographié sur colonne de silice, élue avec des mélanges pentane/éther de polarité croissante (100/0 à 70/30) pour fournir le 2-hydroxy-3-trifluorométhane sulfonate naphtalène.
Rendement : 20%
Formule brute : C₁₁H₇O₃SF₃
Masse molaire : 292,230 g
Point de fusion : 62-63°C.

### Etape 4

Le 5,5-diméthyl-2-(2,2'-bithien-2-yl)[1,3,2]dioxoborinane est préparé suivant le mode opératoire de l'étape 3 de l'exemple 2.

### Etape 5

Le 2-(2,2'-bithien-5-yl)-3-hydroxynaphtalène est obtenu suivant le même protocole expérimental que celui décrit dans l'étape 4 de l'exemple 2.

Sont utilisés ici 11,34 mmoles de l'ester boronique (étape 4 ci-dessus), 9,45 mmoles du naphtol préparée lors de l'étape 3 ci-dessus, 14,17 mmoles de K₃PO₄, une quantité catalytique de Pd (PPh₃)₄ et 13 ml de DMF.
Rendement : 74%
Formule brute : C₁₈H₁₂OS₂
Masse molaire : 308,329 g
Point de fusion :174 °C

### Etape 6

Le 3,5-di(2,2'-bithien-5-yl)-3-(4-pentyloxyphényl)[3H]-naphto[2,1-b]pyrane est obtenu selon le mode opératoire décrit dans l'étape 7 de l'exemple 1.

Les précurseurs sont ici l'alcool propargylique et le naphtol décrits respectivement dans les étapes 2 et 5 de cet exemple 8.
Rendement : 76%
Formule brute : C₄₀H₃₂O₂S₄
Masse molaire : 672,702 g
Point de fusion :77 °C.

### Exemple 8

Des solutions 10⁻⁴ M dans le toluène des composés photochromiques obtenus dans les exemples et les exemples comparatifs ont été préparées.

Les caractéristiques photochromiques ont été déterminées à 20°C sur un spectrophotomètre d'absorption électronique du type Beckman DU 700. Une irradiation externe, perpendiculaire au faisceau d'analyse, était fournie par une lampe à arc Xenon de 150 W équipée d'un filtre à eau, d'un diaphragme et d'un guide de lumière. Le flux lumineux était maintenu constant pendant tous les enregistrements.

Lors d'une première expérience, le spectre d'absorption de la forme colorée obtenue après 2 minutes d'irradiation était enregistré pour déterminer la longueur d'onde maximale d'absorption dans le visible (λmax).

Dans une deuxième expérience, a été enregistrée la variation de l'absorbance de la solution au λmax d'absorption en fonction du temps d'irradiation, le maximum d'absorbance obtenu après quelques secondes à quelques dizaines de secondes d'irradiation a été mesuré (A∞). L'irradiation a été alors arrêtée. La décroissance de l'absorbance en fonction du temps était alors enregistrée permettant d'évaluer la constante de décoloration thermique (kΔ).

Les résultats sont indiqués dans le tableau I ci-dessous.

**TABLEAU I**

| Solvant toluène, T = 20°C | (A∞) | kΔ(s⁻¹) | λmax (nm) FO |
|---|---|---|---|
| Exemple 1 | 0,43 | 0,19 | 549 |
| Exemple 2 | 0,36 | 0,18 | 549 |
| Exemple 3 | 0,38 | 0,19 | 548 |
| Exemple 4 | 0,35 | 0,29 | 564 |
| Exemple 5 | 0,21 | 0,55 | 560 |
| Exemple 6 | 0,50 | 0,19 | 564 |
| Comp. exemple 1 | 0,15 | 0,60 | 520 |
| Comp. exemple 2 | 0,23 | 0,06 | 432 |
| Comp. exemple 3 | 0,13 | 0,24 | 480 |

Les résultats montrent que les composés photochromiques selon l'invention ont une longueur d'onde d'absorption maximale (λmax) très sensiblement déplacée vers les longueurs d'onde élevées (déplacement bathochrome) et un maximum d'absorbance élevé associé à une cinétique rapide.

### Exemple 9

Des solutions toluéniques 10⁻⁴ M des composés photochromiques obtenus dans l'exemple 1 et dans l'exemple comparatif 2 ont été irradiées dans les conditions de l'exemple 8. On a cette fois mesuré les temps de coloration (temps nécessaire pour atteindre l'équilibre photostationnaire) et le pourcentage de décoloration à 10 s après la fin de l'irradiation.

| | Coloration | Décoloration |
|---|---|---|
| Composé | Temps nécesaire pour atteindre l'équilibre photostationnaire (s) | % de décoloration à 10 s |
| Exemple 1 | 14 | 83 % |
| Exemple comparatif 2 | 26 | 50 % |

### Exemple 10

Des solutions toluéniques 10⁻⁴ M des composés photochromiques obtenus dans l'exemple 1 et dans l'exemple comparatif 2 ont été irradiées dans les conditions de l'exemple 8, mais en plaçant dans le faisceau lumineux un filtre GG400 (Schott) coupant tout le rayonnement UV de longueur d'onde inférieure à 380 nm.

Alors que la solution préparée à partir de l'exemple comparatif 2 ne développe pas de coloration, la solution préparée à partir du composé de l'exemple 1 maintient des propriétés photochromiques.

### Exemple 11

Un film de polyuréthane (épaisseur 15 µm) contenant 2.10⁻⁵ ml/g du naphtopyrane de l'exemple 1 a été préparé. Dans les mêmes conditions d'irradiation que celles décrites dans l'exemple 7, cet échantillon a montré d'excellentes propriétés photochromiques :
A∞ = 0,1 , λmax = 344 nm, k = 0.14 s⁻¹

Bien que ralentie par rapport à la solution toluénique de l'exemple 7, la vitesse de décoloration reste très rapide.

## Revendications

1. Composé photochromique possédant une structure [3H] naphto[2,1-b]pyrane, **caractérisé en ce qu'**il comporte au moins un groupement bithiényle, substitué ou non, dans l'une des positions 5 à 10 du cycle naphtalénique de la structure [3H]naphto[2,1-b]pyrane et au moins un groupement bithiényle ou terthiényle, substitué ou non, en position 3 de ladite structure et à l'exclusion des composés de structure [3H]naphto [2,1-b]pyrane comportant un seul groupement bithiényle en position 3 de ladite structure et un groupement bithiényle en position 7 du cycle naphtalénique.

2. Composé selon la revendication 1, **caractérisé en ce qu'**il comprend un groupement bithiényle dans l'une des positions 5, 6, 8-10 du cycle naphtalénique.

3. Composé photochromique selon la revendication 1 ou 2, **caractérisé en ce qu'**il répond à la formule : 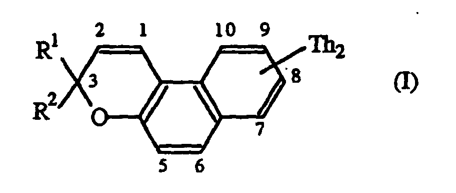 dans laquelle le groupement Th₂ représente un groupement bithiényle répondant à la formule : 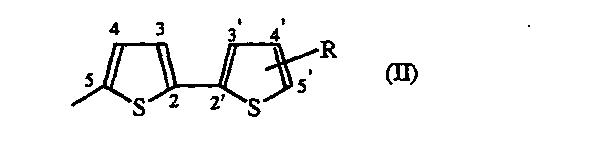 dans laquelle :
R désigne un substituant en positions 3, 3', 4, 4' ou 5', choisi parmi un atome d'hydrogène, un groupement alkyle, un groupement cycloalkyle, un groupement aryle, un groupement OR³, SR³, COR³ et COOR³, où R³ désigne un atome d'hydrogène, un groupement alkyle, un groupement aryle, un groupement amino, un groupement NO₂, CN ou SCN, un atome d'halogène, ou un groupement mono ou polyhaloalkyle;
R¹ désigne un groupement Th₂ tel que défini ci-dessus ou un groupement Th₃ de formule : 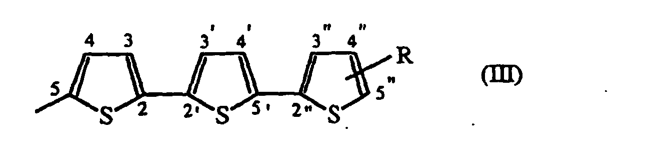
où
R est un substituant tel que désigné ci-dessus pouvant occuper l'une des positions 3, 3', 3", 4, 4', 4", 5" ; et
R² désigne un groupement Th₂ tel que défini dans la formule (II), un groupement Th₃ tel que défini dans la formule (III), un groupement aryle, un groupement naphtyle, un groupement thiényle, un groupement furanyle, un groupement pyrrolyle ou un groupement N-alkylpyrrolyle.

4. Composé photochromique selon la revendication 3, **caractérisé en ce que** les groupements alkyle sont des groupements alkyle en C₁-C₆, les groupements cycloalkyle sont des groupements cycloalkyle en C₅-C₇, les groupements aryle comprennent le phényle et les (C₁-C₆)alkylphényle, et les halogènes sont le brome, le chlore et le fluor.

5. Composé photochromique selon la revendication 3 ou 4, **caractérisé en ce que** dans le groupement Th₂, le substituant R est en position 5'.

6. Composé photochromique selon l'une quelconque des revendications 3 à 5, **caractérisé en ce que** dans le groupement Th₃, le substituant R est en position 5".

7. Composé photochromique selon l'une quelconque des revendications 3 à 6, **caractérisé en ce que** le groupement Th₂ est en position 8 du cycle naphtalénique.

8. Composé photochromique selon l'une quelconque des revendications 3 à 7, **caractérisé en ce que** R¹ est un groupement Th₃.

9. Procédé de préparation d'un composé photochromique selon la revendication 3, **caractérisé en ce qu'**il comprend la réaction, en présence d'un catalyseur acide, d'un alcool propargylique de formule : 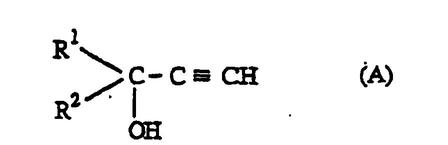 où R¹ et R² sont définis comme dans la revendication 3, avec un 2-naphtol de formule : 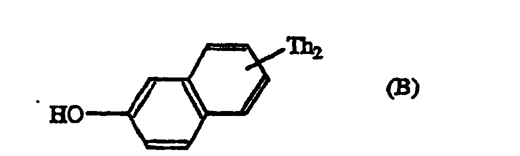 où Th₂ est défini comme dans la revendication 3 et occupe une des positions 3, 4, 6-8 du cycle naphtalénique.

10. Procédé selon la revendication 9, **caractérisé en ce que** le 2-naphtol de formule (B) est préparé par réaction de couplage catalysée par un catalyseur au palladium entre un dérivé boronique du bithiophène et un 2-naphtol substitué sur l'une des positions 3, 4, 6-8 du cycle naphténique par un atome de brome ou un groupement triflate , le dérivé boronique du bithiophène répondant à la formule :

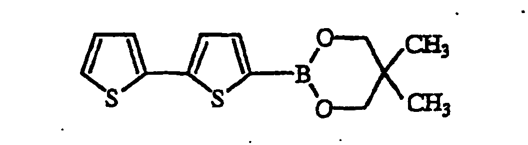

11. Article transparent comprenant un substrat en matériau polymère transparent, **caractérisé en ce qu'**au moins un composé photochromique, selon l'une quelconque des revendications 1 à 8, est incorporé dans le substrat.

12. Article transparent comprenant un substrat en matériau polymère transparent, **caractérisé en ce qu'**au moins une face de l'article est revêtue d'un film en matériau polymère transparent incorporant au moins un composé photochromique selon l'une quelconque des revendications 1 à 8.

13. Article selon la revendication 11 ou 12 **caractérisé en ce que** l'article est un vitrage de bâtiment, un pare-brise d'automobile on d'avion, une visière de casque ou un verre de lunettes.

## Claims

1. Photochromic compound having a [3H]naphtho[2,1-b]pyran structure **characterized in that** it comprises at least one substituted or unsubstituted bithienyl group in one of the positions 5-10 of the naphthalenic ring of [3H]naphtho[2,1-b]pyran structure and at least one substituted or unsubstituted bithienyl or terthienyl group in position 3 of said structure and excluding [3H]naphtho[2,1-b]pyran structure compounds having only a bithienyl group in position 3 of said structure and a bithienyl group in position 7 of the naphthalenic ring.

2. Compound according to claim 1, **characterized in that** it comprises a bithienyl group in one of the positions 5, 6, 8-10 of the naphthalenic ring.

3. Photochromic compound according to claim 1 or 2, **characterized in that** it has the formula: 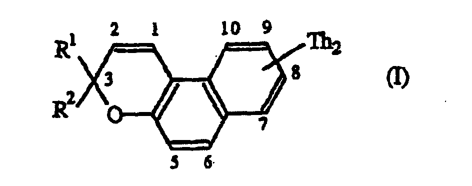 in which the Th₂ group represents a bithienyl group having the formula: 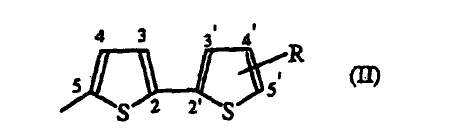 in which:
R is a substituent in positions 3, 3', 4, 4' or 5', selected from a hydrogen atom, an alkyl group, a cycloalkyl group, an aryl group, a group OR³, SR³, COR³ and COOR³, in which R³ is a hydrogen atom, an alkyl group, an aryl group, an amino group, an NO₂, CN or SCN group, a halogen atom or a mono- or polyhaloalkyl group;
R¹ is a Th₂ group as defined above or a Th₃ group of formula: 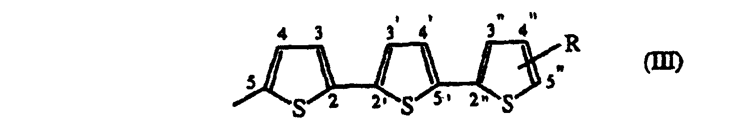 in which
R is a substituent as mentioned above having one of the positions 3, 3', 3", 4, 4', 4", 5"; and
R² is a Th₂ group as defined in formula (II), a Th₃ group as defined in formula (III), an aryl group, a naphthyl group, a thienyl group, a furanyl group, a pyrrolyl group or a N-alkylpyrrolyl group.

4. Photochromic compound according to claim 3, **characterized in that** the alkyl groups are C₁-C₆ alkyl groups, the cycloalkyl groups are C₅-C₇ cycloalkyl groups, the aryl groups comprise phenyl and (C₁-C₆)alkylphenyl groups, and the halogens are bromine, chlorine and fluorine.

5. Photochromic compound according to claim 3 or 4, **characterized in that** in the Th₂ group, the substituent R is in position 5'.

6. Photochromic compound according to anyone of claims 3 to 5, **characterized in that** in the Th₃ group, the substituent R is in position 5".

7. Photochromic compound according to anyone of claims 3 to 6, **characterized in that** the Th₂ group is in position 8 of the naphthalenic ring.

8. Photochromic compound according to anyone of claims 3 to 7, **characterized in that** R¹ is a Th₃ group.

9. Method for preparing a photochromic compound according to claim 3, **characterized in that** it comprises the reaction, in the presence of an acid catalyst, of a propargyl alcohol of formula: 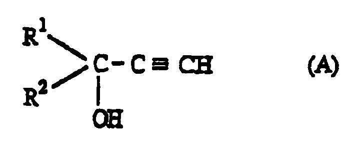 in which R¹ and R² are defined as in claim 3, with a 2-naphthol of formula: 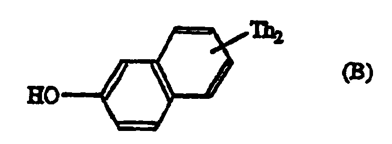 in which Th₂ is defined as in claim 3 and is in one of the positions 3, 4, 6-8 of the naphthalenic ring.

10. Method according to claim 9, **characterized in that** the 2-naphthol of formula (B) is prepared by a coupling reaction in the presence of a palladium catalyst between a bithiophene boronic derivative and a substituted 2-naphthol in one of the positions 3, 4, 6-8 of the naphthalenic ring by a bromine atom or a triflate group, the bithiophene boronic derivative having the formula:

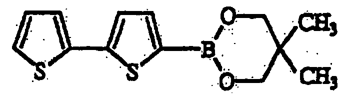

11. Transparent article comprising a substrate made of transparent polymer material, **characterized in that** at least one photochromic compound, according to anyone of claims 1 to 8, is incorporated into the substrate.

12. Transparent article comprising a substrate made of transparent polymer material, **characterized in that** at least one face of the article is coated with a film made of transparent polymer material incorporating at least one photochromic compound according to anyone of claims 1 to 8.

13. Article according to claim 11 or 12, **characterized in that** the article is glazing for the construction industry, a motor vehicle or airplane windshield, a helmet visor or a spectacle glass.

## Patentansprüche

1. Photochromatische Zusammensetzung, die eine [3H]naphtho[2,1-b]pyran-Struktur aufweist, **dadurch gekennzeichnet, dass** die Zusammensetzung zumindest eine Bithienyl-Gruppierung umfasst, die in einer der Positionen 5 bis 10 des Naphthalinringes der [3H]naphtho[2,1-b]pyran-Struktur substituiert ist oder nicht, und zumindest eine Bithienyl oder Terthienyl-Gruppierung, die in Position 3 der genannten Struktur substituiert ist oder nicht, und umfassend ausschließlich der Bestandteile mit [3H]naphtho[2,1-b]pyran-Struktur eine einzelne Bithienyl-Gruppierung in der Position 3 der Struktur und eine Bithienyl-Gruppierung in Position 7 des Naphthalinringes.

2. Zusammensetzung nach Anpruch 1, **dadurch gekennzeichnet, dass** die Zusammensetzung eine Bithienyl-Gruppierung in einer der Positionen 5, 6, 8-10 des Naphthalinringes umfasst.

3. Photochromatische Zusammensetzung nach Anspruch 1 oder 2, **dadurch gekennzeichnet, dass** es der Gleichung genügt: 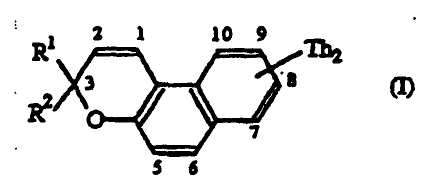 wobei die Gruppierung Th₂ eine Bithienyl-Gruppierung darstellt, der Gleichung genügend: 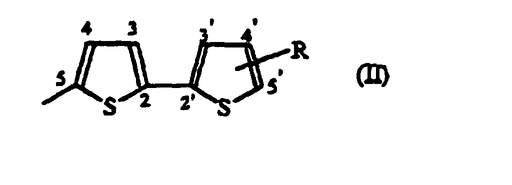 wobei:
R einen Substituenten in den Positionen 3, 3', 4,4' oder 5' beziffert, ausgewählt unter einem Wasserstoffatom, einer Alkylgruppe, einer Cycloalkylgruppe, einer Arylgruppe, einer OR³-, SR³-, COR³- und einer COOR³-Gruppierung, wobei R³ ein Wasserstoffatom beziffert, eine Alkylgruppe, eine Arylgruppe, eine Aminogruppe, eine NO₂-, CNoder SCN-Gruppe, ein Halogenatom, oder eine Mono- oder Polyhaloalkylgruppe;
R¹ eine Gruppe oder Gruppierung Th₂ beziffert, wie oben definiert, oder eine Gruppierung Th₃ mit der Gleichung: 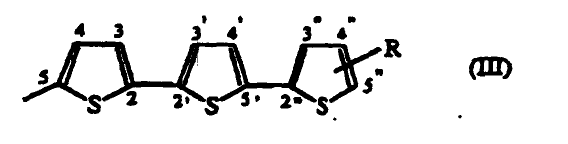
wobei R ein Substituent ist, wie vorangehend angegeben, in der Lage eine der Positionen anzunehmen unter 3, 3', 3", 4, 4', 4", 5"; und
wobei R² eine Th₂-Gruppierung, wie in Gleichung (II) definiert beziffert, eine Gruppierung Th₃, wie in Gleichung (III) definiert, eine Arylgruppierung, eine Naphthylgruppierung, eine Thienylgruppierung, eine Furanylgruppierung, eine Pyrrolylgruppierung oder eine N-Alkylpyrrolylgruppierung.

4. Photochromatische Zusammensetzung nach Anspruch 3, **dadurch gekennzeichnet, dass** die Alkylgruppierungen Alkylgruppierungen in C₁ bis C₆ sind, die Cycloalkylgruppierungen Cycloalkylgruppierungen in C₅ bis C₇ sind, die Arylgruppierungen Phenyl- und (C₁-C₆)-Alkylphenyl umfassen und die Halogene Brom, Chlor und Fluor sind.

5. Photochromatische Zusammensetzung nach Anspruch 3 oder 4, **dadurch gekennzeichnet, dass** in der Th₂-Gruppierung der Substituent R in der Position 5' vorliegt.

6. Photochromatische Zusammensetzung nach einem der Ansprüche 3 bis 5, **dadurch gekennzeichnet, dass** in der Th₃-Gruppierung, der Substituent R in der Position 5" vorliegt.

7. Photochromatische Zusammensetzung nach einem der Ansprüche 3 bis 6, **dadurch gekennzeichnet, dass** die Th₂-Gruppierung in Position 8 des Naphthalinrings vorliegt.

8. Photochromatische Zusammensetzung nach einem der Ansprüche 3 bis 7, **dadurch gekennzeichnet, dass** R¹ eine Th₃-Gruppierung ist.

9. Verfahren zur Herstellung einer photochromatischen Zusammensetzung nach Anspruch 3, **dadurch gekennzeichnet, dass** es die Reaktion umfasst, im Beisein eines sauren Katalysators, eines Propargylalkohols mit der Gleichung: 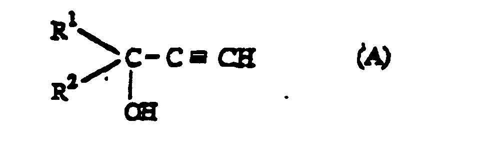 wobei R¹ und R² definiert sind wie im Anspruch 2, mit einem 2-Naphthol der Gleichung: 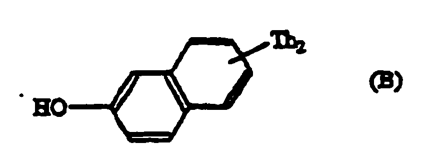 wobei Th₂ wie im Anspruch 3 definiert ist und eine der Positionen 3, 4, 6-8 des Naphthalinringes einnimmt.

10. Verfahren nach Anspruch 9, **dadurch gekennzeichnet, dass** das 2-Naphthol der Gleichung (B) dargestellt wird mittels katalytischer Kopplungsreaktion mittels eines Katalysators mit Palladium zwischen einem Boron-Derivat des Bithiophens und einem 2-Naphthol, welches an einer der Positionen 3, 4, 6-8 des Naphthalinrings substituiert ist, durch einen Bromatom oder eine Triflatgruppierung, wobei das Borderivat des Bithiophens der Gleichung genügt:

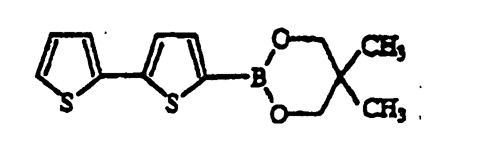

11. Durchsichtiger oder transparenter Gegenstand, umfassend ein Substrat aus transparentem Polymermaterial, **dadurch gekennzeichnet, dass** zumindest eine photochromatische Zusammensetzung gemäß einem der Ansprüche 1 bis 8 in das Substrat eingebettet ist.

12. Durchsichtiger Gegenstand, umfassend ein Substrat aus transparentem Polymermaterial, **dadurch gekennzeichnet, dass** zumindest eine Fläche des Gegenstandes mit einem Film beschichtet ist aus transparentem Polymermaterial, umfassend zumindest eine photochromatische Zusammensetzung nach einem der Ansprüche 1 bis 8.

13. Gegenstand nach Anspruch 11 oder 12, **dadurch gekennzeichnet, dass** der Gegenstand eine Gebäudeverglasung, eine Kraftfahrzeug- oder Flugzeugwindschutzscheibe, ein Helmvisier oder ein Brillenglas ist.
